# EUROPEAN PATENT SPECIFICATION

(11) **EP 2 185 221 B1**
(45) Date of publication and mention of the grant of the patent: **11.12.2013**
(21) Application number: 08830524.8
(22) Date of filing: 15.09.2008
(51) Int. Cl.: A61M 5/178, A61M 5/00

(54) **INFUSION AND TRANSFER SYSTEM FOR USE WITH RADIOACTIVE AGENTS**
INFUSIONS- UND ÜBERTRAGUNGSSYSTEM ZUR VERWENDUNG MIT RADIOAKTIVEN WIRKSTOFFEN
PERFUSION ET SYSTÈME DE TRANSFERT POUVANT ÊTRE UTILISÉS AVEC DES AGENTS RADIOACTIFS

(30) Priority: 13.09.2007 US 972001 P
(43) Date of publication of application: 19.05.2010
(73) Proprietor: Molecular Insight Pharmaceuticals, Inc., Cambridge, MA 02142 (US)
(72) Inventor: LAFRANCE, Norman, Cambridge, MA 02142 (US); DE LA GUARDIA, Miguel, Lincoln Park New Jersey 07035 (US)
(74) Representative: Murphy, Colm Damien
(86) International application number: PCT/US2008/076426
(87) International publication number: WO 2009/036443

(56) References cited:
- EP-A- 1 616 587
- EP-A- 1 769 811
- EP-A- 1 820 730
- WO-A-2005/118031
- JP-A- 2000 350 783
- US-A- 4 401 108

## Description

### BACKGROUND

Radiopharmacology is the study and preparation of radiopharmaceuticals, *i.e*., radioactive pharmaceuticals. Radiopharmaceuticals are used in the field of nuclear medicine as tracers in the diagnosis and treatment of many diseases.

Radiotherapy can also be delivered through infusion (into the bloodstream) or ingestion. Examples are the infusion of metaiodobenzylguanidine (MIBG) to treat neuroblastoma, of oral iodine-131 to treat thyroid cancer or thyrotoxicosis, and of hormone-bound lutetium-177 and yttrium-90 to treat neuroendocrine tumors (peptide receptor radionuclide therapy). Another example is the injection of radioactive glass or resin microspheres into the hepatic artery to radioembolize liver tumors or liver metastases.

Radiolabeled macromolecules have also been and are being developed. Radioimmunotherapeutic agents, for example, FDA-approved Ibritumomab tiuxetan (Zevalin), which is a monoclonal antibody anti-CD20 conjugated to a molecule of Yttrium-90, Tositumomab Iodine-131 (Bexxar), which conjugates a molecule of Iodine-131 to the monoclonal antibody anti-CD20, were the first radioimmunotherapy agents approved for the treatment of refractory non-Hodgkin's lymphoma.

Although radiolabeled agents are being developed and are increasingly more effective at treating particular diseases and disorders, they involve certain risks, especially to health care professionals, and especially when required in large doses. Improved methods and devices are needed for the delivery of radiolabeled therapeutics.

Prior art document EP1769811 discloses a chemical liquid injection system whereby a chemical liquid in a chemical liquid container can be directly injected into a patient without using an intervening liquid syringe or the like. A leading end of liquid supply tube is placed at an upper position within the chemical liquid container and a trailing end of the liquid supply tube is connected to the liquid container. The liquid pressure-feeding mechanism feeds the liquid within the liquid container into the chemical liquid container from liquid supply tube.

Prior art document US 4401108 relates to syringe loading shields for use with syringes during the drawing, calibration and injection of aliquots of radioactive materials. Each syringe loading shield is provided with means for shielding radiation emanating from the mouth of the vial during the material-withdrawal, syringe loading process.

### SUMMARY

Described herein are infusion systems and methods for delivering a radiopharmaceutical agent to a subject, such that an administering health care professional does not get exposed to a potentially deleterious amount of radiation. The systems and methods described herein allow for combined, *i.e.,* increased radiation doses to be delivered to the subject. The infusion and transfer systems of the present invention can be used to deliver any radiopharmaceutical agent that has a potentially deleterious amount of radiation.

One embodiment is directed to a dose delivery infusion system, comprising: at least one first reservoir containing a radiopharmaceutical agent with a cannula inserted into the reservoir and a valve that connects the cannula to a second reservoir; and a radiation shield surrounding the at least one first reservoir; characterized in that venting unit is connected to at least one first reservoir. In another embodiment, the at least one first reservoir is a vial containing the radiopharmaceutical agent. In one embodiment, the vial comprises a slanted bottom. In another embodiment, the radiation shield is lead. In one embodiment, the second reservoir is attached to an infusion pump. In another embodiment, the agent is a radiopharmacological agent labeled with an isotope selected from the group consisting of: Technetium-99m (technetium-99m), Iodine-123 and 131, Thallium-201, Gallium-67, Yttrium-90, Samarium-153, Strontium-89, Phosphorous-32, Rhenium-186, Fluorine-18 and Indium-111. In one embodiment, the radiopharmaceutical agent is selected from the group consisting of: Bexxar^{®} (Iodine I-131 Tositumomab), Zevalin^{®} (Yttrium Y-90 Ibritumomab Tiuxetan), Quadramet^{®} (Samarium Sm-153 Lexidronam), Strontium-89 chloride, Phosphorous-32, Rhenium-186 hydroxyethlidene, Samarium-153 lexidronam, I-131 Iobenguane (Azedra^{®}), Y-90 edotreotide (Onalta^{®}) and an I-131 labeled benzamide (Solazed^{®}).

We also describe herein a method for delivering an effective dose of a radiopharmaceutical agent, comprising, infusing the radiopharmaceutical agent using a system comprising: at least one first reservoir containing a radiopharmaceutical agent with a cannula inserted into the reservoir and a airtight connector that connects the cannula to a second reservoir; and a radiation shield surrounding the at least one first reservoir. At least one first reservoir is a vial containing the radiopharmaceutical agent. The vial may comprise a slanted bottom. The system used in the method may further comprise a filtered vent connected to the at least one first reservoir. The radiation shield may be lead. The second reservoir may be attached to an infusion pump. The radiopharmaceutical agent may be selected from the group consisting of: Bexxar^{®} (Iodine I-131 Tositumomab), Zevalin^{®} (Yttrium Y-90 Ibritumomab Tiuxetan), Quadramet^{®} (Samarium Sm-153 Lexidronam), Strontium-89 chloride, phosphorous-32, rhenium-186 hydroxyethlidene, samarium-153 lexidronam, I-131 Iobenguane, Y-90 edotreotide and an I-131 labeled benzamide.

### BRIEF DESCRIPTION OF THE DRAWINGS

FIG. 1 shows a schematic of an I-131 Iobenguane (MIBG) therapeutic infusion system in accordance with an embodiment of the present invention.
FIG. 2 shows a schematic of an I-131 Iobenguane (MIBG) therapeutic dose transfer system in accordance with an embodiment of the present invention.

### DETAILED DESCRIPTION

Described herein are apparatus systems and methods for administering radiolabeled compounds to a subject such that, for example, greater than about 700 mCi can be delivered in a manner that does not expose health care professionals to a hazardous radiation dose. As used herein, the term "subject" refers to an animal. The animal can be a mammal, *e.g*., either human or non-human. A subject can be, for example, primates (*e.g*., monkeys, apes and humans), cows, pigs, sheep, goats, horses, dogs, cats, rabbits, rats, mice, fish, birds and the like. As used herein, the term "dose" refers to an effective amount of a therapeutic agent. Doses can be measured in, for example, any measure of quantity including, for example, a unit for measuring radioactive dose. Doses are known for known therapeutic agents, and, if not known, one of skill in the art would be able to determine an effective amount of a therapeutic agent. As used herein, the term "efficacy" refers to the degree to which a desired effect is obtained, and an "effective amount" is an amount sufficient to produce a desired therapeutic effect.

### Background

Nuclear medicine is a branch of medicine and medical imaging that uses the nuclear properties of matter in diagnosis and therapy. It produces images that reflect biological processes that take place at the cellular and subcellular level.

Nuclear medicine procedures use pharmaceuticals that have been labeled with radionuclides (radiopharmaceuticals). In diagnosis, radioactive substances are administered to patients and the radiation emitted is detected. The diagnostic tests involve the formation of an image using a gamma camera or positron emission tomography. Imaging may also be referred to as radionuclide imaging or nuclear scintigraphy. Other diagnostic tests use probes to acquire measurements from parts of the body, or counters for the measurement of samples taken from the patient.

In therapy, radionuclides are administered to treat disease or provide palliative pain relief. For example, administration of Iodine-131 is often used for the treatment of thyrotoxicosis and thyroid cancer. Phosphorus-32 was formerly used in treatment of polycythemia vera. Those treatments rely on the killing of cells by high radiation exposure, as compared to diagnostics in which the exposure is kept as low as reasonably achievable (ALARA policy) so as to reduce the chance of inducing a cancer.

### Diagnostic testing

Diagnostic tests in nuclear medicine exploit the way that the body handles substances differently when there is disease or pathology present. The radionuclide introduced into the body is often chemically bound to a complex that acts characteristically within the body; this is commonly known as a tracer. In the presence of disease, a tracer will often be distributed around the body and/or processed differently. For example, the ligand methylene-diphosphonate (MDP) can be preferentially taken up by bone. By chemically attaching technetium-99m to MDP, radioactivity can be transported and attached to bone via the hydroxyapatite for imaging. Any increased physiological function, such as due to a fracture in the bone, will usually mean increased concentration of the tracer. This often results in the appearance of a 'hot-spot', which is a focal increase in radio-accumulation, or a general increase in radio-accumulation throughout the physiological system. Some disease processes result in the exclusion of a tracer, resulting in the appearance of a 'cold-spot'. Many tracer complexes have been developed to image or treat many different organs, glands, and physiological processes.

### Types of studies

A typical nuclear medicine study involves administration of a radionuclide into the body by intravenous injection in liquid or aggregate form, ingestion while combined with food, inhalation as a gas or aerosol, or rarely, injection of a radionuclide that has undergone micro-encapsulation. Some studies require the labeling of a patient's own blood cells with a radionuclide (leukocyte scintigraphy and red blood cell scintigraphy). Most diagnostic radionuclides emit gamma rays, while the cell-damaging properties of beta particles are used in therapeutic applications. Refined radionuclides for use in nuclear medicine are derived from fission or fusion processes in nuclear reactors, which produce radioisotopes with longer half-lives, or cyclotrons, which produce radioisotopes with shorter half-lives, or take advantage of natural decay processes in dedicated generators, *i.e*., molybdenum/technetium or strontium/rubidium. Commonly used intravenous radionuclides include, but are not limited to:
Technetium-99m (technetium-99m)
Iodine-123 and 131
Thallium-201
Gallium-67
Fluorine-18
Indium-111

### Radiation dose

A patient undergoing a nuclear medicine procedure will receive a radiation dose. Under present international guidelines, it is assumed that any radiation dose, however small, presents a risk. The radiation doses delivered to a patient in a nuclear medicine investigation present a very small risk of inducing cancer. In this respect, it is similar to the risk from X-ray investigations except that the dose is delivered internally rather than from an external source such as an X-ray machine. As discussed above, health care professionals, although exposed to much lower radiation does, are also at risk because of their exposure to the multiple administrations of radiation to numerous patients.

The radiation dose from a nuclear medicine investigation is expressed as an effective dose with units of sieverts (usually given in millisieverts, mSv). The effective dose resulting from an investigation is influenced by the amount of radioactivity administered in megabecquerels (MBq), the physical properties of the radiopharmaceutical used, its distribution in the body and its rate of clearance from the body.

Effective doses can range from 6 µSv (0.006 mSv) for a 3 MBq chromium-51 EDTA measurement of glomerular filtration rate to 37 mSv for a 150 MBq thallium-201 non-specific tumor imaging procedure. The common bone scan with 600 MBq of technetium-99m-MDP has an effective dose of 3 mSv.

Other units of measurement include the Curie (Ci), being 3.7E10 Bq, and also 1.0 grams of Radium (Ra-226); the Rad (radiation absorbed dose), now replaced by the Gray; and the Rem (Rad Equivalent Man), now replaced with the Sievert. The Rad and Rem are essentially equivalent for almost all nuclear medicine procedures, and only alpha radiation will produce a higher Rem or Sv value, due to its much higher Relative Biological Effectiveness (RBE).

### Radiopharmaceutical Agents

A radiopharmaceutical agent can be any agent that requires infusion for administration to a subject for a diagnostic or therapeutic purpose. A radiopharmaceutical dose is measured in the amount of radiation delivered, *e.g*., mCi. The system and methods described herein can deliver a dose of, for example, >700 mCi, about 200 mCi to about 700 mCi, about 250 mCi to about 500 mCi, or about 300 mCi to more than about 700 mCi.

The systems and methods described herein can be used to deliver any radiopharmaceutical agent to a subject, including, but not limited to, a radiopharmacological agent labeled with an isotope selected from the group consisting of: Technetium-99m (technetium-99m), Iodine-123 and 131, Thallium-201, Gallium-67, Yttrium-90, Samarium-153, Strontium-89, Phosphorous-32, Rhenium-186, Fluorine-18 and Indium-111. Examples of radiopharmaceutical agents that can delivered using the present invention include, but are not limited to, Bexxar^{®} (Iodine I-131 Tositumomab), Zevalin^{®} (Yttrium Y-90 Ibritumomab Tiuxetan), Quadramet^{®} (Samarium Sm-153 Lexidronam), Strontium-89 chloride, phosphorous-32, rhenium-186 hydroxyethlidene, samarium-153 lexidronam, I-131 Iobenguane, Y-90 edotreotide or an I-131 labeled benzamide. The systems and methods described herein are infusion systems and methods that reduce the amount of potentially deleterious radiation exposure otherwise experienced by, for example, a health care professional or patient.

The infusion systems and methods described herein allow for the combination of one or more vials containing a radiopharmaceutical agent, thereby allowing for the infusion delivery of an increased dose to the patient, without exposing a health care professional to a harmful level of radiation.

### Dosage Delivery and Infusion System

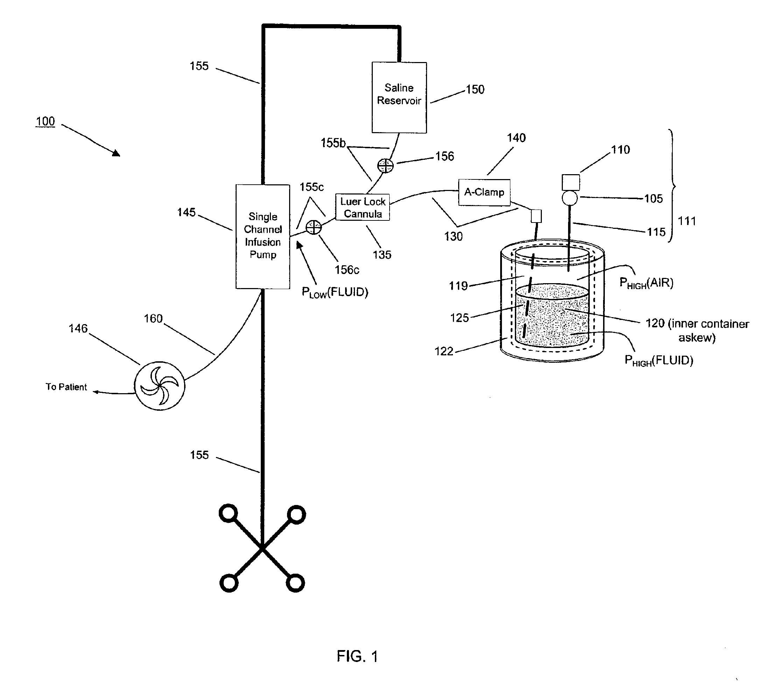

FIG. 1 shows a schematic of a radiopharmaceutical agent, e.g., I-131 Iobenguane (MIBG), infusion system in accordance with an embodiment of the present invention. In some embodiments, the therapeutic infusion system 100 includes a 0.22 µm syringe filter 105 that is attached to a charcoal filter unit 110 and to a 20G × 1" Luer Lock needle 115 at the opposite end of the charcoal filter unit 110. The unit, including the 0.22 µm syringe filter 105, the charcoal filter unit 110, and the 20G × 1" Luer Lock needle 115, make up a venting unit 111. The venting unit 111 is inserted into a patient dose vial 120. In some embodiments, the patient dose vial 120 includes a 100 mL sterile vial. In some embodiments, there can be more than one dose vial, thereby allowing for multiplying the dose for the infusion system. In some embodiments, the patient dose vial(s) 120 includes a slanted bottom, and is askew to a lead (Pb) shield 122. The lead shield 122 prevents one or more radioactive elements contained in the patient dose vial 120 from contaminating one or more of operators and a patient.

In some embodiments, a 19G × 3.5" aspirating needle 125 is attached to a secondary line 130 and to a Luer Lock cannula 135 at the opposite end of the 19G × 5" aspirating needle 125. In some embodiments, the secondary line 130 is a 24" male-male (M-M) arterial pressure tubing. In some embodiments, an A-clamp 140 is clamped to the secondary line 130, initially inhibiting fluid flow between the 19G × 5" aspirating needle 125 and the Luer Lock cannula 135. The Luer Lock cannula 135 is inserted into a primary tubing injection site above a single channel infusion pump 145. In some embodiments, the A-clamp 140 is flushed prior to clamping the secondary line 130.

In some embodiments, the Luer Lock cannula 135 is also attached to a normal saline reservoir 150. An infusion pump primary line 155b and 155c connects the normal saline reservoir 150, supported above the patient dose vial 120 by an intravenous (IV) stand 155, to the primary tubing injection site above the single channel infusion pump 145.

In some embodiments, the A-clamp 140 is open, the primary line check valve 156 is closed, and the primary line check valve 155c is open. As such, the lowered pressure at the primary tubing injection site above the single channel infusion pump 145 pulls a fluid from the patient dose vial 120 through the secondary line 130, the Luer Lock cannula 135, and an infusion pump primary line 155c and into the single channel infusion pump 145, and on to a patient through an infusion pump delivery line 160. The venting unit 111 prevents a pressure equalization between the primary tubing injection site above the single channel infusion pump 145 and the patient dose vial 120, which would inhibit the fluid flow from the patient dose vial 120.

In some embodiments, a setting on the single channel infusion pump 145 can set an infusion rate for the fluid from the patient dose vial 120. In some embodiments, a fill volume in the patient dose vial 120 is 50 mL, and a recommended infusion rate is 100 mL per hour. In some embodiments, the infusion will occur over a 30 minute period at the recommended infusion rate. In some embodiments, an infusion rate can be set by an in-line flow regulator valve with a locking wheel 146. In some embodiments, the in-line flow regulator valve with a locking wheel 146 may be in one of the primary line 155c, the secondary line 130, and the infusion pump delivery line 160. In some embodiments, the fluid from the patient dose vial 120 is I-131 Iobenguane.

In some embodiments, the A-clamp 140 is then closed and the primary line check valve 156 is opened. As such, the lowered pressure at the primary tubing injection site above the single channel infusion pump 145 pulls a saline solution from the saline reservoir 150 through the primary lines 155b and 155c, the primary line check valves 156 and 155c, and the Luer Lock cannula 135, and into the single channel infusion pump 145, effectively flushing the primary lines 155b and 155c of the fluid from the patient dose vial 120.

In some embodiments, the A-clamp 140 is then opened and the primary line check valve 155c is closed. As such, the height differential between the saline reservoir 150 and the patient dose vial 120 allows the saline solution from the saline reservoir 150 to flow through the primary line 155b, the Luer Lock cannula 135, and secondary line 130 into the patient dose vial 120, effectively flushing the secondary line 130 of the fluid from the patient dose vial 120. In some embodiments, the saline reservoir 150 consists of at least 50 mL of a 0.9% NaCl solution.

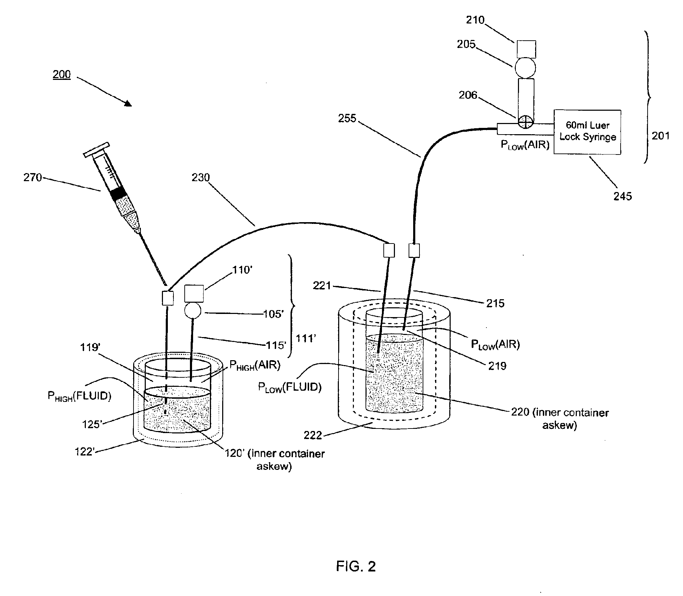

FIG. 2 shows a schematic of a radiopharmaceutical dose transfer system in accordance with an embodiment of the present invention. The radiopharmaceutical dose transfer system 200 allows for transferring a fluid from a shipping vial 120' to a sealed patient dose vial 220. In some embodiments, the dose transfer system 200 includes a 0.22 µm syringe filter 105', a charcoal filter unit 110', and a 20G × 1" Luer Lock needle 115', which collectively make up a venting unit 111' analogous to the venting unit 111 described previously for the radiopharmaceutical infusion system 100 given in FIG. 1. Additionally, the radiopharmaceutical dose transfer system 200 includes a shipping vial 120' and a 19G × 3.5" aspirating needle 125' analogous to the patient dose vial 120 and the 19G × 3.5" aspirating needle 125 described previously for the radiopharmaceutical infusion system 100 given in FIG. 1. The analogous elements in systems 100 and 200 represent an identical method in which the fluid is extracted from the patient dose vial 120 in system 100 and the shipping vial 120' in system 200.

The venting unit 111' is inserted into the shipping vial 120'. The venting unit 111' keeps an ambient pressure in the shipping vial head 119', resulting in an ambient fluid pressure in the shipping vial 120'. In some embodiments, the shipping vial 120' includes a 30 mL sterile vial. In some embodiments, the shipping vial 120' includes a slanted bottom, and is askew to a lead shield 122'. The lead shield 122' prevents one or more radioactive elements contained in the shipping vial 120' from contaminating one or more of operators and a patient.

In some embodiments, the radiopharmaceutical dose transfer system 200 further includes a transfer tubing set 201, which includes a 0.22 µm syringe filter 205 attached to a charcoal filter unit 210, a three-way stopcock valve 206 attached to the opposite end of the 0.22 µm syringe filter 205, and a 60 mL Luer Lock syringe 245. Drawing back the plunger of the 60 mL Luer Lock syringe 245 with the three-way stopcock valve 206 closed creates a vacuum in a primary air line 255 and a sealed patient dose vial head 219 of the sealed patient dose vial 220. In some embodiments, the sealed patient dose vile 220 seal is a 20G × 1" Luer Lock needle 215, connected directly to the three-way stopcock valve 206 and sealed at the sealed patient dose vile 220. The reduced pressure in the sealed patient dose vial head 219 results in a reduced fluid pressure in the sealed patient dose vial 220.

The differential pressure between the fluid in the shipping vial 120' and the fluid in the sealed patient dose vial 220 pulls the fluid in the shipping vial 120' through the 19G × 3.5" aspirating needle 125', a secondary line 230, and a 20G × 1.5" Luer Lock needle 221 into the sealed patient dose vial 220. The venting unit 111' prevents a pressure equalization between the fluid in the shipping vial 120' and the fluid in the sealed patient dose vial 220 by pinning the pressure of the fluid in the shipping vial 120' to the ambient pressure.

In some embodiments, the primary air line 255 is a 48" extension set male-female (M-F) connector. In some embodiments, the secondary line 230 is a 12" arterial pressure tubing male-male (M-M) connector.

In some embodiments, the sealed patient dose vial 220 includes a 100 mL sterile vial. In some embodiments, the sealed patient dose vial 220 includes a slanted bottom, and is askew to a lead (Pb) shield 222. The Pb shield 222 prevents one or more radioactive elements contained in the sealed patient dose vial 220 from contaminating one or more of operators and a patient.

In some embodiments, following a fluid transfer from the shipping vial 120' to the sealed patient dose vial 220, the three-way stopcock valve 206 is opened and the plunger of the 60 mL Luer Lock syringe 245 is depressed to equalize the pressure in the sealed patient dose vial head 219 to the ambient pressure and to remove excess air from the primary air line 255.

In some embodiments, the radiopharmaceutical dose transfer system 200 can provide multiple dosing levels by repeating the previously outlined steps for system 200 without replacing the sealed patient dose vial 220. In some embodiments, following completion of the previously outlined steps for the radiopharmaceutical dose transfer system 200, the dose itself may require a fine adjustment.

In some embodiments, the fine adjustment may be made to the radiopharmaceutical dose by placing the shipping vial 120' on its side inside its Pb shield 122' and, using a shielded 10 mL syringe 270 with a 20G × 1.5" needle, removing a volume from the shipping vial 120' necessary to achieve a prescribed dose, and transferring the contents of the syringe 270 volume to the sealed patient dose vial 220. In some embodiments, the pressure in the sealed patient dose vial 220 may be reduced from a positive pressure resulting from the fluid transfer to an ambient pressure by pulling back the plunger of the syringe 270 to remove an equal volume of air from the sealed patient dose vial 220.

Similarly, in some embodiments a required volume of sterile water may be added to the sealed patient dose vial 220 using the shielded 10 mL syringe 270 with the 20G × 1.5" needle, removing a volume from a sterile water vial (not shown) necessary to achieve a prescribed total volume, and transferring the sterile water contents of the syringe 270 volume to the sealed patient dose vial 220. In some embodiments, the pressure in the sealed patient dose vial 220 may be reduced from a positive pressure resulting from the fluid transfer to an ambient pressure by pulling back the plunger of the syringe 270 to remove an equal volume of air from the sealed patient dose vial 220.

### Example: I-131 Iobenguane Dosage Delivery and Infusion System

The recommended I-131 Iobenguane (Azedra^{®} Drug Delivery System can deliver a therapeutic dose to a subject. Described herein is an I-131 Iobenguane Drug Delivery System and procedures for use.

I-131 Iobenguane is used for the treatment of metastatic neuroendocrine tumors such as pheochromocytoma, carcinoid and neuroblastoma that are not amenable to treatment with surgery or conventional chemotherapy. The I-131 Iobenguane Drug Product consists of an MIBG molecule radiolabeled by chemically binding to a radioactive Iodine isotope through Ultratrace^{®} technology. The iodine isotope acts either diagnostically for imaging disease or therapeutically to deliver targeted radiation to the tumor site. I-131 Iobenguane incorporates an iodine isotope, targets specific tumor cells and does not contain unwanted carrier molecules, or cold contaminants. Cold contaminants are avoided using our proprietary Ultratrace^{®} technology.

### Clinical Overview:

I-131 Iobenguane has received Orphan Drug status and a Fast Track designation by the FDA. A Phase I dosimetry trial was completed and was designed to evaluate the safety, tolerability and distribution of I-131 Iobenguane in adult patients with one of two forms of neuroendocrine cancer (e.g., cardinal or pheochromocytoma).

The primary objective for the I-131 Iobenguane Phase I portion is designed to determine the maximum tolerated dose (MTD) of Ultratrace^{®} lobenguane I-131. The Phase II portion is designed to show that Ultratrace^{®} lobenguane I-131 monotherapy administered at the MTD found in the phase I study is safe and effective for refractory high-risk pheochromocytoma/neuroblastoma.

### Drug Delivery System

Described herein is an overview and description of the recommended apparatus and its intended use, as well as a guideline for sites to use when purchasing commercially available components and assembling the apparatus for delivery of the I-131 Iobenguane product, although it will be appreciated that the dose delivery and infusion system can be readily applied to any radiopharmaceutical agent.

The recommended I-131 Iobenguane Drug Delivery System consists of the following configurations: 1) Therapeutic Infusion System and 2) Therapeutic Dose Transfer System, refer to attached schematics, component lists and guidelines for use.
4.1 MIP Therapeutic System Schematics
   4.1.1 Therapeutic Infusion System
   4.1.2 Therapeutic Dose Transfer System
4.2 Component Lists
4.3 MIP Working Practice Guidelines
   4.3.1 Therapeutic Dose Transfer Process
   4.3.2 Therapeutic Infusion System

### I-131 Iobenguane (I-131 MIBG) Therapeutic Infusion System Working Practice Guideline

1. Obtain operating IV access. Preferred IV access sites: Bilateral forearms (non-dominant side recommended), bilateral hands (not wrists). If preferred IV access sites are unobtainable, elbow and wrist access is possible but immobilization of the extremity is strongly recommended to avoid extravasation of the site. A central line is also acceptable.
2. Hang a 250 mL bag of 0.9% Sodium Chloride Solution for Injection, USP and spike the bag using the infusion pump primary set and prime the tubing.
3. Attach the primary line to the IV access site on the patient.
4. For the primary line (0.9% Sodium Chloride) the recommended rate is 100 mL per hour. Allow the primary line to run for at least 30 minutes.
5. Attach 0.22 µm syringe filter to the charcoal filter unit and attach a 20G × 1" needle to the opposite end of the filter. Insert the whole unit into the patient dose vial.
6. Attach the 19G × 5" aspirating needle to 24" M-M arterial pressure tubing. To the other end of the tubing attach the Luer Lock cannula.
7. Attach an A-clamp to the arterial pressure line and clamp it completely. Insert the cannula into primary tubing injection site above the pump.
8. Flush the arterial line by releasing the A-clamp. Clamp the line once it has been flushed.
9. Line up the 5" aspirating needle with the line on the pig and insert it into the patient dose vial at a slight angle. CAUTION: 5" Aspirating needle can catch side of plastic vial. Ensure that 5" needle has reached the bottom of the vial.
10. Using the piggyback setting on the pump, set infusion rate of I-131 Iobenguane (I-131 MIBG). The fill volume in the patient dose vial is 50 mL, and the recommended rate is 100 mL per hour. The infusion of I-131 Iobenguane (I-131 MIBG) will occur over 30 minutes at the recommended rate.
11. Using an A-clamp, clamp the primary line slightly above the secondary line injection site.
12. Remove the A-clamp on the arterial pressure tubing (secondary line). Begin the infusion by watching the arterial pressure tubing to make sure I-131 Iobenguane (I-131 MIBG) is being administered.
13. After 25 minutes, watch for air bubbles in the arterial pressure tubing. Once the first air bubbles form in the arterial pressure tubing clamp off the tubing.
14. Remove the clamp from the primary line and flush the remaining volume of I-131 Iobenguane in the primary line with at least 50 mL of 0.9% Sodium Chloride to administer residual drug.
15. Unclamp the secondary tubing and to allow the 0.9% Sodium Chloride solution to flush any residual I-131 Iobenguane (I-131 MIBG) in the in the secondary tubing back into the patient dose vial.
16. Clamp the primary tubing near the patient when the 0.9% Sodium Chloride flush is complete and detach the patient from the IV tubing.
17. Return used dose vial to radiopharmacy and measure residual activity and record on CRP.

### I-131 Iobenguane (I-131 MIBG) Therapeutic Dose Transfer Protocol

1. Determine the activity required for a patient dose based on the dosing protocol and patient's weight; add 5% to account for loss in administration.
2. Insert the empty 100 mL patient dose vial into a lead shield and swab the septum top and the septum of the shipping vials in the warming shields with an alcohol swab.
3. Assemble the "venting unit" by attaching the male end of a charcoal filter to the female end of a 0.22 µm filter and 20G × 1" needle to the male end of the filter.
4. Insert the "venting unit" into an I-131 Iobenguane shipping vial.
5. Assemble the "transfer tubing set" by attaching a 20G × 1" needle to the male end of the 48" M-F extension set and the female end to the 3-way stopcock.
6. To the female "T" port of the stopcock, attach a 0.22 µm filter and to the female end of the filter attach a charcoal filter.
7. To the in-line female port of the 3-way stopcock attach an empty 60 mL syringe.
8. Insert the 20G × 1" needle of the "transfer tubing set" into the empty 100 mL patient dose vial.
9. Attach a 20G × 1.5" needle to the 12" M-M arterial pressure tubing and connect the opposite end of the line to the 19G × 3.5" aspirating needle.
10. Insert the 20G × 1.5" needle Into the patient dose vial and insert the 19G × 3" aspirating needle into the shipping vial to the bottom of the angled warming shield (*i.e.,* tip of aspirating needle at the lowest point insides the vial)
11. Draw back the plunger of the 60 mL syringe to vacuum transfer the solution from the I-131 Iobenguane shipping vial to the patient dose vial.
12. Close the 3-way stopcock to the patient dose vial line (open to the charcoal trap). Depress the plunger of the 60 mL syringe to push out the air. Open the patient dose vial line on the 3-way stopcock.
13. If more than ONE VIAL is needed to fulfill the patient dose repeat steps 4-9.
14. Remove the needles from the patient dose vial and assay in a dose calibrator.
15. Calculate additional mL required to adjust the patient dose to the prescribed activity (add 5% for loss during administration).
16. Place the unused shipping vial inside its lead shield on its side and using a shielded 10 mL syringe with a 20G × 1.5" needle, remove the volume necessary to achieve the prescribed dose. Add the syringe contents to the patient dose vial. Pull back the plunger of the syringe to remove an equal volume of air from the patient dose vial to avoid a state of positive pressure in the vial.
17. Based on the volume (mL) of drug transferred to the patient dose vial, calculate the amount of Sterile Water for Injection, USP needed to QS the dose to a final volume of 50 mL.
18. Using an empty 60 mL syringe with a 20G × 1" needle, draw up the required volume of Sterile Water for Injection, USP and add it to the patient dose vial. Pull back the plunger of the syringe to remove an equal volume of air from the patient dose vial to avoid a state of positive pressure in the vial.
19. Remove all the needles from the patient dose vial and measure the radioactivity in a radionuclide dose calibrator to verify the patient dose.
20. Record the patient dose on the case report form.

The present disclosure is not to be limited in terms of the particular embodiments described in this application, which are intended as illustrations of various aspects.

While various aspects and embodiments have been disclosed herein, other aspects and embodiments will be apparent to those skilled in the art.

## Claims

1. A dose delivery infusion system (100), comprising:
at least one first reservoir (120) containing a radiopharmaceutical agent with a cannula (135) inserted into the reservoir;
a valve (156) that connects the cannula (135) to a second reservoir (150);
a radiation shield (122) surrounding the at least one first reservoir (120); and
**characterized in that** a venting unit (111) is connected to the at least one first reservoir (120).

2. The dose delivery system of Claim 1, wherein the at least one first reservoir (120) is a vial containing the radiopharmaceutical agent.

3. The dose delivery system of Claim 2, wherein the vial (120) comprises a slanted bottom.

4. The dose delivery system of Claim 1, wherein the radiation shield (122) is lead.

5. The dose delivery system of Claim 1, wherein the second reservoir (150) is attached to an infusion pump (145).

6. The dose delivery system of Claim 1, wherein the radiopharmaceutical agent is selected from the group consisting of: Bexxar^{®} (Iodine I-131 Tositumomab), Zevalin^{®} (Yttrium Y-90 Ibritumomab Tiuxetan), Quadramet^{®} (Samarium Sm-153 Lexidronam), Strontium-89 chloride, phosphorous-32, rhenium-186 hydroxyethlidene, samarium-153 lexidronam, I-131 Iobenguane, Y-90 edotreotide and an I-131 labeled benzamide.

## Patentansprüche

1. Dosisverabreichungs- und Infusionssystem (100), das aus Folgendem besteht:
mindestens einem ersten Behälter (120), der ein radiopharmazeutisches Mittel enthält, und einer im Behälter eingesetzten Kanüle (135);
einem Ventil (156), das die Kanüle (135) mit einem zweiten Behälter (150) verbindet;
einen Strahlungsschutz (122), der den mindestens einen ersten Behälter (120) umgibt, und
**dadurch gekennzeichnet, dass** am mindestens einen ersten Behälter (120) eine Belüftungsvorrichtung (111) angeordnet ist.

2. Das Dosisverabreichungs- und Infusionssystem nach Anspruch 1, wobei der mindestens eine erste Behälter (120) eine Ampulle ist, die ein radiopharmazeutisches Mittel enthält.

3. Das Dosisverabreichungs- und Infusionssystem nach Anspruch 2, wobei die Ampulle (120) eine abgeschrägte Unterseite aufweist.

4. Das Dosisverabreichungs- und Infusionssystem nach Anspruch 1, wobei der Strahlungsschutz (122) aus Blei besteht.

5. Das Dosisverabreichungs- und Infusionssystem nach Anspruch 1, wobei der zweite Behälter (150) mit einer Infusionspumpe (145) verbunden ist.

6. Das Dosisverabreichungs- und Infusionssystem nach Anspruch 1, wobei das radiopharmazeutische Mittel aus der Gruppe bestehend aus Bexxar^{®} (Iod-131 Tositumomab), Zevalin^{®} (Yttrium-90-Ibritumomab-Tiuxetan), Quadramet^{®} (Samarium-153-Lexidronam), 89-Strontiumchlorid, Phosphor-32, Rhenium-186- Hydroxyethyliden, Samarium-153-Lexidronam, I-131-Iobenguan, Y-90-Edotreotid und einem I-131-markiertes Benzamid ausgewählt ist.

## Revendications

1. Système d'administration de dose par perfusion (100), comprenant :
au moins un premier réservoir (120) contenant un agent radiopharmaceutique avec une canule (135) introduite dans le réservoir ;
un obturateur (156) reliant la canule (135) à un deuxième réservoir (150) ;
un écran de protection radiologique (122) entourant l'au moins un premier réservoir (120) ; et
**caractérisé en ce qu'**un module de ventilation (111) est relié à l'au moins un premier réservoir (120).

2. Système d'administration de dose selon la revendication 1, dans lequel l'au moins un premier réservoir (120) est un flacon contenant l'agent radiopharmaceutique.

3. Système d'administration de dose selon la revendication 2, dans lequel le flacon (120) comporte un fond incliné.

4. Système d'administration de dose selon la revendication 1, dans lequel l'écran de protection radiologique (122) est en plomb.

5. Système d'administration de dose selon la revendication 1, dans lequel le deuxième réservoir (150) est fixé à une pompe de perfusion (145).

6. Système d'administration de dose selon la revendication 1, dans lequel l'agent radiopharmaceutique est sélectionné parmi le groupe composé de : Bexxar^{®} (Iode I-131 tositumomab), Zevalin^{®} (yttrium Y-90 ibritumomab tiuxetan), Quadramet^{®} (samarium Sm-153 lexidronam), chlorure de strontium-89, phosphore-32, rhénium-186 hydroxyéthylidène, samarium-153 lexidronam, 1-131 iobenguane, Y-90 edotreotide et un benzamide marqué au I-131.
